Europäisches Patentamt

European Patent Office

Office européen des brevets

(11) Veröffentlichungsnummer: **0 451 309 A1**

## (12) EUROPÄISCHE PATENTANMELDUNG

(21) Anmeldenummer: **90106818.9**

(22) Anmeldetag: **10.04.90**

(51) Int. Cl.5: **A61B 17/30, B25G 1/10**

(43) Veröffentlichungstag der Anmeldung:
**16.10.91 Patentblatt 91/42**

(84) Benannte Vertragsstaaten:
**AT BE CH DE DK ES FR GB GR IT LI LU NL SE**

(71) Anmelder: **S & T Marketing AG**
**Zollstrasse 91**
**CH-8212 Neuhausen(CH)**

(72) Erfinder: **Vogel, Max**
**Zollstr. 91**
**CH-8212-Neuhausen am Rheinfall(CH)**

(74) Vertreter: **Hiebsch, Gerhard F., Dipl.-Ing. et al**
**Erzbergerstrasse 5A Postfach 464**
**W-7700 Singen 1(DE)**

(54) **Chirurgisches Instrument mit Griffteil.**

(57) Chirurgisches Instrument, insbesondere mikro-chirurgische Schere oder Pinzette (10) mit Griffteil und wenigstens einer sich über zumindest einen Abschnitt des Griffteils erstreckender, gegenüber den benachbarten Instrumentenabschnitten reibungs-erhöhter Greifoberfläche (18) wird dadurch verbes-sert, daß die Greifoberfläche (18) als Aufrauhung mit kleinen, nicht nebeneinander angeordneten Erhebungen und Vertiefungen unterschiedlicher Bemessung und in der Bemessung unregelmäßiger Anordnung ausgebildet ist.

Fig.1

EP 0 451 309 A1

Die Erfindung betrifft ein chirurgisches Instrument gemäß dem Gattungsbegriff des Patentanspruches 1.

Bei chirurgischen Instrumenten wie Pinzetten, Gefäßdilatatoren, Klemmanlegepinzetten, Scheren, Nadelhaltern, Arteriotomieklemmen usw. wird zwischen Flach- und Rundgriffinstrumenten unterschieden; bei einem Flachgriffinstrument, beispielsweise bei einer Pinzette deren Schenkel ist das Griffteil im Querschnitt rechteckig, bei einem Rundgriffinstrument jedoch halbkreisförmig ausgebildet.

In der Entwicklung chirurgischer Instrumente, insbesondere für mikrochirurgische Zwecke, besteht der Zwang, diese laufend ergonomisch zu verfeinern; die Zielsetzung geht dahin, die Hand des Operateurs während der Arbeit zu entlasten und den Bedienungskomfort zu steigern. Dazu wurden beispielsweise bei Flachgriffinstrumenten die Griffteile an ihrer ihren äußeren Oberfläche/n mit in Längsrichtung verlaufenden Greifoberflächen ausgestattet, deren Länge sich aus operationstechnischen Abstandsveränderungen der Chirurgenhand zum Operationsfeld bestimmt. Zur sicheren Handhabung der Instrumente weisen die Greifoberflächen - die sich im Falle von Flachgriffinstrumenten zumeist über die gesamte Breite des Griffteiles erstrecken, während sie beim Rundgriffinstrument auf der Umfangsfläche verlaufen -- unterschiedliche Rauhungen in Form von Löchern oder von zur Längsachse der Griffteile quer verlaufenden spanabhebend eingearbeiteten Rillen auf, an denen sich eine Fingerbeere des Benutzers während der Betätigung des Instumentes abzustützen vermag.

Insbesondere bei Rundgriffinstrumenten ist es bekannt, auf deren Umfang sich kreuzende Rinnen vorzusehen, so daß je nach Rinnenabstand quadratische oder rechteckige Oberflächenerhebungen entstehen.

Mit derartigen Greifoberflächenausbildungen wurde durch besondere Konstruktion der Instrumente eine Gleichgewichtslage in der Hand des Chirurgen geschaffen und ermöglicht, vermittels Gewichtsreduktionen und gegebenenfalls unter verwendungsspezifischer Feinabstimmung des Druckes von Federelementen die Handmuskelkraft des Operateurs zu schonen. Einen hohen Arbeitskomfort vermittelnde mikrochirurgische Instrumente dieser Art wurden von der Anmelderin entwickelt und sind als Stand der Technik in deren der Öffentlichkeit zugänglichen Verkaufsunterlagen beschrieben.

Bei Operationen mittlerer und längerer Dauer, insbesondere solcher mikrochirurgischer Art, sind Gleichgewichtslage, geringes Gewicht und minimiert eingestellte Federdrücke nach Erkenntnis der Erfinder nicht allein maßgebend, durch entsprechende Reduzierung der dadurch sonst in Anspruch genommenen Muskelkraft, Ermüdungserscheinungen der Hand eines Chirurgen zu vermindern und in ihrem Anftreten zurückzudrängen.

Umfang und Eintrittszeit von Ermüdungserscheinungen bestimmen vielmehr auch die Höhe der lokalen Druckbelastung der Fingerbeere und damit einhergehend ein mehr oder minder ausgeprägtes Druckempfinden, die bei Instrumenten der vorstehend beschriebenen Art auch nicht durch mechanische Feinstbearbeitung, wie Rundung, Politur und Beschichtung der Rillen und Oberflächenerhebungen und durch Tragen chirurgischer Handschuhe zu eliminieren sind.

Hiervon ausgehend hat sich der Erfinder die Aufgabe gestellt, chirurgische Instrumente der eingangs erwähnten Art zu schaffen, bei denen die Höhe der lokalen Druckbelastung und das Druckempfinden auf die Fingerbeere im Vergleich zu den bekannten Griffflächen reduziert und eine sichere Handhabung des Instrumentes gewährleistet ist.

Zur Lösung dieser Aufgabe führt, daß die Greifoberfläche als Aufrauhung mit kleinen, dicht nebeneinander angeordneten Erhebungen und Vertiefungen unterschiedlicher Bemessung und in der Bemessung unregelmäßiger Anordnung ausgebildet ist. Dabei soll die Rauhtiefe vorteilhafterweise weniger als 0,5 mm, bevorzugt weniger als 0,2 mm betragen.

Nach weiteren Merkmalen der Erfindung soll die Oberfläche durch mechanische Aufrauhung oder durch chemische Aufrauhung oder durch elektrische Aufrauhung gebildet sein; eine spanabhebende Oberflächenbearbeitung erfüllt nicht die vom Erfinder gesehene Aufgabe.

Im Rahmen der Erfindung liegt es, den für die Greifoberfläche vorgesehenen Abschnitt des Griffteils zu entfetten, dann aufzurauhen und mit Aluminiumoxid auf dem Wege des Plasmaspritzens zu beschichten.

Griffflächenausbildungen der erfindungsgemäßen Art setzen überraschenderweise den Druck auf die Fingerbeere und das Druckempfinden während der Benutzung eines chirurgischen Instrumentes deutlich herab, womit Ermüdungserscheinungen merklich verkleinert und zweitlich weiter verzögert werden.

Weitere Vorteile, Merkmale und Einzelheiten der Erfindung ergeben sich aus der nachfolgenden Beschreibung eines bevorzugten Ausführungsbeispiels sowie anhand der Zeichnung; diese zeigt in:

Fig. 1: eine Draufsicht auf eine mikrochirurgische Pinzette mit einer erfindungsgemäß ausgebildeten Greifoberfläche;

Fig. 2: einen überhöhten Schnitt durch Fig. 1 nach deren Linie II - II.

Griffflächen der folgend beschriebenen und beanspruchten Art können selbstverständlich auch an den Griffteilen anderer chirurgischer Instrumente, wie Gefäßdilatatoren, Klemmanlegepinzetten, Scheren, Nadelhaltern, Arteriotomieklemmen und Haltern von Gegendruckschlingen zur Anwendung kommen.

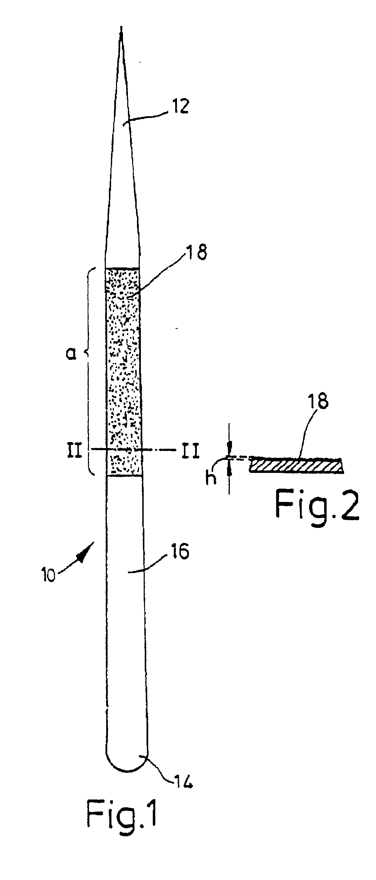

In Fig. 1 ist eine Pinzette 10 in der Draufsicht als Flachgriffinstrument mit einer Spitze 10 und einem ein gerundetes Ende 14 anbietenden Griffteil 16 wiedergegeben. An die in Draufsicht konische Spitze 10 schließt eine aufgerauhte Greifoberfläche 18 an, die sich quer über das gesamte Griffteil 16 erstreckt und von einer Länge a ist. Die Oberflächenstruktur der Greifoberlfäche weiset kleine, dicht nebeneinander angeordnete Erhebungen und Vertiefungen unterschiedlicher Bemessung und in der Bemessung unregelmäßiger Anordnung auf. Das Maß der maximalen Rauhtiefe ist in Fig. 2 mit h bezeichnet und mißt etwa 0,2 mm.

Diese Rauhigkeit wird durch mechanische, chemische oder elektrische Aufrauhung erzeugt. Die mechanische Aufrauhung erfolgt mittels Granulatstrahlen wie Glasstrahlung, die chemische durch Ätzen, während die elektrische mittels Funkenerosion durchführbar ist.

Bei diesen Aufrauhungsverfahren bleiben Größe und Höhe h der Erhebungen bzw. Vertiefungen sowie ihre Anordnung und Verteilung in Abhängigkeit vom verwendeten Granulat, der Säure und Ätzdauer bzw. gemäß Oberflächenkonfiguration der Erosionselektrode variabel, so daß für jedes chirurgisches Instrument bestimmungsgemäß eine bestmögliche Oberflächenstruktur einstellbar ist.

**Patentansprüche**

1. Chirurgisches Instrument, insbesondere mikrochirurgische Schere oder Pinzette, mit Griffteil und wenigstens einer sich über zumindest einen Abschnitt des Griffteils erstreckender, gegenüber den benachbarten Instrumentenabschnitten reibungserhöhter Greifoberfläche,

   dadurch gekennzeichnet,

   daß die Greifoberfläche (8) als Aufrauhung mit kleinen, nicht nebeneinander angeordneten Erhebungen und Vertiefungen unterschiedlicher Bemessung und in der Bemessung unregelmäßiger Anordnung ausgebildet ist.

2. Chirurgisches Instrument nach Anspruch 1, dadurch gekennzeichnet, daß die Oberfläche durch mechanische Aufrauhung gebildet ist.

3. Chirurgisches Instrument nach Anspruch 1, dadurch gekennzeichnet, daß die Oberfläche durch chemische Aufrauhung gebildet ist.

4. Chirurgisches Instrument nach Anspruch 1, dadurch gekennzeichnet, daß die Oberfläche durch elektrische Aufrauhung gebildet ist.

5. Chirurgisches Instrument nach Anspruch 1, dadurch gekennzeichnet, daß die Greifoberfläche (8) durch einen thermischen Spritzvorgang hergestellt ist.

6. Chirurgisches Instrument nach Anspruch 5, dadurch gekennzeichnet, daß die Greifoberfläche (8) aus Aluminiumoxid besteht.

7. Chirurgisches Instrument nach einem der Ansprüche 1 bis 6, gekennzeichnet durch eine Rauhtiefe (h) von weniger als 0,5 mm, bevorzugt unter 0,2 mm.

8. Chirurgisches Instrument nach einem der Ansprüche 1 bis 7, gekennzeichnet durch eine Rauhtiefe (h) von 0,1 mm.

Fig.2

Fig.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-4 693 246  (H.G. REIMELS)<br>* Anspruch 1; Spalte 2, Zeile 66 - Spalte 3, Zeile 10; Figur 1 *<br>– – – | 1,2-4,7,8 | A 61 B 17/30<br>B 25 G 1/10 |
| Y | GB-A-2 086 792  (MICROSURGICAL ADMINISTRATIVE SERVICES LTD.)<br>* Ansprüche 1-7,9-13; Figuren 1-6 *<br>– – – | 1,2-4,7,8 | |
| Y | DE-U-8 406 785  (M. NEUMAYR)<br>* Ansprüche 1-3; Figur *<br>– – – | 3 | |
| A | FR-A-2 506 295  (ROSENTHAL AG)<br>* Ansprüche 1,4,7; Figur 1 *<br>– – – | 6 | |
| A | CH-B-3 774 79  (F. KELLER)<br>* Anspruch 1; Seite 1, Zeilen 16-25,35-61; Figuren 1-3 *<br>– – – – – | 1,3-5 | |

| RECHERCHIERTE SACHGEBIETE (Int. Cl.5) |
|---|
| A 61 B<br>B 25 G |

Der vorliegende Recherchenbericht wurde für alle Patentansprüche erstellt

| Recherchenort | Abschlußdatum der Recherche | Prüfer |
|---|---|---|
| Berlin | 30 November 90 | PAPA E.R. |